# EUROPEAN PATENT APPLICATION

(11) **EP 4 151 162 A1**
(43) Date of publication of application: **22.03.2023**
(21) Application number: 21805052.4
(22) Date of filing: 10.05.2021
(51) Int. Cl.: A61B 17/12

(54) **UMBILICAL ANCHOR SYSTEM**

(30) Priority: 11.05.2020 ES 202030426
(71) Applicant: FUNDACIÓN PARA LA INVESTIGACIÓN BIOMÉDICA DEL HOSPITAL 12 DE OCTUBRE, 28041 Madrid (ES)
(72) Inventor: TAJUELO LLOPIS, Imanol, 28053 Madrid (ES)
(74) Representative: ZBM Patents - Zea, Barlocci & Markvardsen
(86) International application number: PCT/ES2021/070324
(87) International publication number: WO 2021/229123

(57) **Abstract**

An anchor system for anchoring an umbilical catheter to an umbilical cord is disclosed. The system comprises a vascular anchor element and an anchor clamp. The vascular anchor element comprises a conduit configured to accommodate a portion of an umbilical catheter, and one end configured to be inserted into a blood vessel of the umbilical cord. The anchor clamp is configured to releasably retain at least one portion of the umbilical cord and at least one portion of the anchor element. The clamp comprises a recess configured to receive the at least one portion of the umbilical cord and the at least one portion of the anchor element; and a releasable closure system for selectively opening/closing the anchor clamp.

## Description

The present description relates to anchor systems, more specifically to anchor systems for umbilical catheters.

### BACKGROUND

The use of umbilical catheters is sometimes necessary for administering treatments to neonatal patients, for example, the administration of certain medications or to perform a transfusion. Such umbilical catheters need to be secured to avoid complications and/or patient injury in case of, for example, accidental displacement of the catheter involving excessive penetration.

The use of different devices that employ adhesive systems to secure the catheter to the patient's abdominal region is well known. However, these devices have disadvantages that may compromise the patient's health.

On the one hand, the current systems make use of adhesive materials that may result in damage to sensitive, i.e. baby, skin of neonatal patients, which may cause abrasions and/or lacerations at the time of removal.

On the other hand, known systems do not allow secure fixation of the catheter since there is the possibility of accidental displacement of the catheter, for example, if the patient tilts and leans against the catheter. Such displacements, whether inward or outward, may cause sequelae, for example, if an unsterile portion penetrates the body this may lead to infection, or in case of excessive penetration, the catheter may damage an internal organ. Furthermore, in case the catheter becomes dislodged, it is necessary to repeat the cannulation process, that is, removing the catheter and inserting it again, which entails time during which the patient does not receive any treatment.

Moreover, current devices do not adequately protect the exposed region of the umbilical cord, which increases the risk of infection and the possibility of bleeding.

In some cases, it is known to supplement the use of a clamping device with a tourniquet at the base of the umbilical cord by tying a silk thread there around or by means of a suture, to prevent bleeding and to provide minimum catheter clamping, although this is neither effective nor safe.

Performing a suture involves risk for both the patient and the healthcare personnel due to the use of sharp tools. On the other hand, in cases where a silk thread is tied, the knot may get loose, losing effectiveness and requiring constant monitoring. Moreover, neither the suture nor the silk thread completely prevents bleeding or accidental displacement of the catheter.

In addition, in order to check that the catheter is in the correct position, i.e. at the correct fixing distance, it is well known to take X-rays. However, in the event that catheter re-cannulation is required, for example, due to accidental mobilization, the patient may be required to be exposed to radiation on repeated occasions, which is highly inadvisable.

In summary, there is a need for a device capable of reliably and securely fixing an umbilical catheter, which avoids or significantly reduces the risk of infection, bleeding, and radiation exposure of the patient, and which, at the same time, is easy to use.

### SUMMARY

In a first aspect, an anchor system for anchoring an umbilical catheter to an umbilical cord is defined. The system comprises a vascular anchor element and an anchor clamp. The vascular anchor element comprises a conduit configured to accommodate a portion of an umbilical catheter, and an end configured to be inserted into a blood vessel of the umbilical cord. The anchor clamp is configured to releasably retain at least one portion of the umbilical cord and at least one portion of the anchor element. The clamp comprises a recess configured to receive the at least one portion of the umbilical cord and the at least one portion of the anchor element, and a releasable closure system for selectively opening/closing the anchor clamp.

The use of a vascular anchor element allows the blood vessel into which the catheter is inserted to be occluded without compromising the function of the catheter, i.e. without altering the flow through the catheter. This reduces the risk of infection and bleeding.

On the other hand, the use of an anchor clamp makes it possible (once it is closed) to occlude the blood vessels that have not been cannulated and to close the exposed part of the umbilical cord. Consequently, bleeding is prevented and the risk of contamination, and thus infection, is avoided or substantially reduced.

In addition, a force is exerted by the anchor clamp (when closed) in the form of pressure on the retained portion of the umbilical cord and also on a portion of the catheter, such that the catheter is firmly anchored. The use of the anchor clamp thus avoids accidental displacements that may result in the catheter changing its position, for example, over-penetrating or pulling out. By preventing unwanted displacements, repetition of cannulation operation, i.e. removing and reinserting of the catheter, is also prevented.

The clamp recess can also allow anchoring of both venous and arterial catheters and even simultaneous anchoring of two vascular catheters in the event that vein and umbilical artery cannulation is required.

On the other hand, by using an anchor system as claimed, performing sutures at the base of the umbilical cord is not required. This provides a safe and easy-to-use system that can be placed quickly and easily and does not require the use of dangerous tools such as needles or scalpels that may cause cuts to the patient and/or healthcare personnel. Furthermore, in order to remove the anchor system, it is sufficient to open the anchor clamp to release the umbilical cord and then remove the fixing element together with the umbilical catheter, allowing removal of the system to be easy, fast, and safe.

Also, the claimed system allows the catheter to be anchored to the umbilical cord, rather than to the abdominal region, so that the use of adhesive materials is not required. In other words, the anchor system does not damage the skin of neonatal patients.

On the other hand, the use of a releasable closure system allows the fixing distance to be changed where required without replacing the entire umbilical anchor system. The releasable closure system makes it possible to release the clamp and adjust or correct the fixing distance of the umbilical catheter, for example, by sliding on the vascular anchor element along the catheter.

Additionally, the claimed anchor system allows the umbilical cord to be viewed, both the insertion point of the catheter, i.e. the exposed area, and the base of the cord, allowing the main points to be viewed to detect symptoms of umbilical cord infection.

In some examples, the anchor clamp may comprise two portions hingedly attached together.

In some examples, the anchor element may comprise a lug configured to removably engage an anchor clamp fixing groove. By using the lug, clamping of the anchor element to the anchor clamp is enhanced, providing a more secure system.

In some examples, the internal shape of the fixing groove may be complementary to the external shape of the lug of the anchor element.

In some examples, the conduit of the anchor element may be configured to allow relative sliding between the catheter and the anchor element, allowing the anchor element to be placed at a particular point in the catheter, for example, for indicating the fixing distance. This facilitates proper placement of the catheter at the appropriate fixing distance, and the healthcare personnel can have a reference point and use the anchor element as a stop. In some examples, the umbilical catheter may comprise a plurality of lines to indicate different fixing distances, further facilitating correct placement and reducing the time required for cannulation.

In some examples, the outer surface of the vascular anchor element may be funnel-shaped to facilitate insertion of the anchor element and occlusion of the blood vessel once inserted therein.

In some examples, the releasable closure system may comprise at least one tab arranged at one portion of the anchor clamp, and a closure groove located at the opposite portion of the anchor clamp, the groove being configured to releasably receive the at least one tab.

In some examples, the releasable closure system may further comprise an actuator for releasing the at least one tab from the closure groove.

In some examples, the end of the vascular anchor element configured to be inserted into a blood vessel of the umbilical cord may comprise an antiseptic coating on the conduit and/or the outer surface to reduce the risk of infection.

In some examples, the coating may be in the form of a ring.

In some examples, the anchor element and the anchor clamp may be a unitary piece.

In some examples, the anchor clamp may comprise an additional fixing element on the outer surface of the anchor clamp.

### BRIEF DESCRIPTION OF THE DRAWINGS

Non-limiting examples of the present description are described below, with reference to the accompanying drawings, wherein:
Figure 1 schematically illustrates a plan view of an anchor system according to one example;
Figure 2 schematically illustrates an anchor element according to one example;
Figure 3 schematically illustrates schematically a cross section of an umbilical cord with an umbilical catheter and an anchor element according to one example;
Figure 4 schematically illustrates an anchor clamp according to one example;
Figure 5 schematically illustrates an anchor system according to one example; and
Figure 6 illustrates a flow chart of a method for anchoring an umbilical catheter to an umbilical cord.

### DETAILED DESCRIPTION OF EXAMPLES

To clarify some of the terms used in the present application, definitions of such terms are included herein below.

By "cannulation" is meant the process of inserting an umbilical catheter into any of the three blood vessels of the umbilical cord, i.e., into the vein or into either of the two arteries.

By "umbilical catheter" is meant both venous and arterial catheters which are used in umbilical blood vessel cannulation.

The "fixing distance" is the length or portion of the catheter that must remain inside the patient. The fixing distance depends on the characteristics of the patient, the treatment to be applied, etc., and varies for each case. The different fixing distances are included in a number of validated tables that may be consulted by healthcare personnel or may be calculated by means of pre-established formulas that take into account the patient's weight. The terms "fixing distance", "proper position" and "correct position" are used interchangeably.

The figures in the present application are not to scale for ease of understanding.

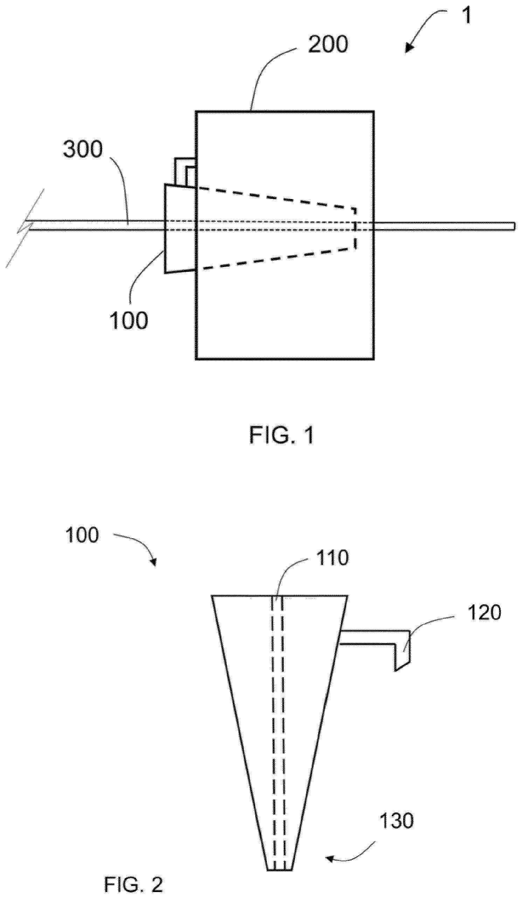

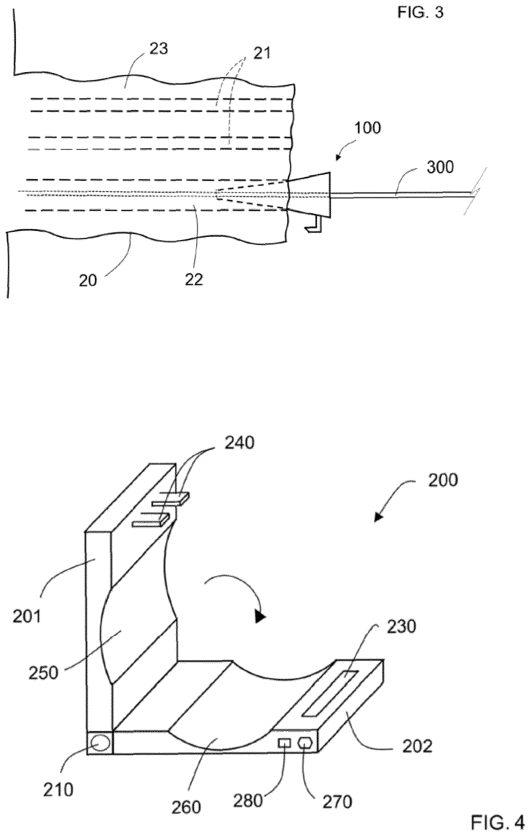

Figure 1 shows an anchor system 1 for anchoring an umbilical catheter to an umbilical cord. The anchor system 1 may comprise a vascular anchor element 100 (see figure 2) and an anchor clamp 200 (see figure 4) configured to releasably retain at least a portion of the vascular anchor element and a portion of the umbilical cord.

Figure 2 shows a vascular anchor element 100 that may comprise a conduit 110 configured to accommodate a portion of an umbilical catheter. The diameter of the conduit may be any diameter as long as the flow in the conduit is not blocked.

The diameter of the conduit 110 may vary depending on the type of catheter being used. For example, if it is necessary to give medication or perform a blood test, the blood vessel to be cannulated is the umbilical vein, so a venous catheter will be required. On the other hand, for measuring blood pressure or performing arterial blood tests, the blood vessel that is cannulated is one of the two umbilical arteries, and therefore an arterial catheter is required. Due to the difference in diameters between the different blood vessels i.e. veins and arteries, the diameter of the venous catheters are larger than that of the arterial catheters. Consequently, the diameter of the venous conduit 110 will be larger in cases where the use of a venous catheter is required. In one example, the diameter of the hole may be about 1 - 2.2 mm, in one example about 1.30 - 1.65 mm. In another example, the diameter of the hole may be about 0.6 - 1.25 mm, in one example about 0.8 - 1.15 mm.

The diameter of the conduit 110 may be configured to facilitate relative sliding between the catheter and the vascular anchor element. In some examples, a small gap may exist between the outer portion of the catheter and the conduit. In additional or alternative examples, the surface of the conduit may be polished, or it may comprise a coating to reduce friction.

Furthermore, in some cases the umbilical catheter may comprise a plurality of markings along its length to indicate a fixing distance with each marking. The vascular anchor element may thus slide along the catheter to the corresponding marking and serve as a guide or stop for the healthcare personnel. That is, the catheter can be inserted until the vascular anchor element is within the cannulated blood vessel.

In some examples the conduit 110 may be configured to allow relative sliding in a single direction. That is, the vascular anchor element is allowed to slide toward one end of the catheter, for example, toward the end to be inserted into the patient's body, but not in the opposite direction.

For this purpose, the conduit may comprise, for example, a plurality of tabs or any other mechanism to provide resistance in one direction. In this way it is possible, for example, to prevent unwanted displacement of the vascular anchor element once it has been positioned to indicate the fixing distance.

In some examples the conduit 110 may be configured to allow relative sliding in both directions.

As shown in the example of figure 2, the outer surface of the vascular anchor element 100 may be funnel-shaped or cone-shaped, i.e., an outer shape whose diameter increases progressively from a narrower end to a wider end. The narrower end of the anchor element may thus facilitate insertion of the vascular anchor element into the blood vessel and the progressively increasing outer shape helps the vessel to be dilated, which reduces the risk of damaging the blood vessel. On the other hand, said blood vessel may be plugged or occluded by the wider end, once inserted, thus preventing bleeding and entry of bacteria into the circulatory system, reducing the risk of infection.

In one example, the dimensions of the vascular anchor element, or more specifically, the dimension of the wider end of the vascular anchor, may be wide enough to plug or occlude veins and arteries interchangeably, for example, by varying the portion of the anchor element being inserted within the blood vessel. The portion of the anchor element inserted within a vessel will be larger in vein cannulation since the diameter of veins is larger than the diameter of arteries. In one example, the wider end of the anchor element may have a diameter equal to or less than 3 mm.

In alternative examples, the dimensions of the vascular anchor element, or more specifically, the dimension of the widest end of the vascular anchoring, will depend on the type of catheter, and therefore, on the type of blood vessel to be cannulated. That is, the anchor element may be of two types: a venous anchor element and an arterial anchor element. In such examples, the wider end of the venous anchor element may be larger than that of the arterial anchor element.

The vascular anchor element 100 may also comprise a lug 120 configured to removably engage an anchor clamp fixing groove which will be described further below. In one example, the lug 120 may be L-shaped or may have any other shape that facilitates engagement and does not protrude to prevent snagging.

The lug may be positioned in the widest area of the anchor element, i.e., in the area intended to remain outside the blood vessel to avoid contact with the umbilical cord once the anchor element is inserted into the blood vessel.

In some examples, the end of the vascular anchor element configured to be inserted into a blood vessel may comprise an antiseptic coating (not shown) to reduce the risk of infection. The coating may be applied to the conduit and/or the outer surface of the anchor element. In one example, the coating may be ring-shaped. In one example, the coating may be silver alginate or any other compound with antiseptic properties.

Sometimes, simultaneous cannulation of two umbilical blood vessels may be required, for example, of the umbilical vein and one of the umbilical arteries. In such cases two anchor elements may be used.

A cross section of an umbilical cord 20 with a vascular anchor element 100 positioned in one of its blood vessels is shown in figure 3. As shown in figure 3, an umbilical cord 20 comprises three blood vessels: a vein 22 and two arteries 21, wrapped in Wharton's jelly 23.

In the figure, the anchor element 100 is inserted into the umbilical vein, so that the vein is occluded while allowing the flow in catheter 300 to remain unaltered, so that in this example the anchor element is a venous anchor element, and the catheter is a venous catheter.

Figure 4 shows an anchor clamp 200 configured to releasably retain at least one portion of the umbilical cord and at least one portion of the anchor element. The anchor clamp 200 may comprise two hingedly attached pieces or portions 201, 202. For this purpose, the anchor clamp may comprise a hinged joint 210 attached to both portions of the clamp that allows opening between the portions and thus facilitates positioning of the umbilical cord between the two portions. In one example (see figure 4), the degree of opening provided by the hinged joint may be at least 90 degrees. In one example, the degree of opening may be at least 180 degrees.

In one example, the portions 201, 202 and the hinged joint 210 may be separate elements. In such examples, the hinged joint may be, for example, a hinge or any pivoting element.

In alternative examples, the portions 201, 202 and the hinged joint 210 may be integral, i.e., they may be made of a single piece. In such examples, the hinged joint may be formed by folds and simulate the shape of a bellows.

Additionally, the anchor clamp 200 may comprise a releasable closure system for selectively opening/closing the anchor clamp. The closure system may comprise at least one tab 240 arranged on one of the portions 201, 202 of the anchor clamp, and a closure groove 230 located at the opposite portion of the anchor clamp and configured to releasably receive the at least one tab.

In the example of figure 4, the anchor clamp 200 comprises two tabs 240.

The closure system may further comprise an actuator 270 for releasing the at least one tab from the closure groove and facilitating opening of the clamp while preventing accidental removal. In some examples (not shown), the closure system may comprise two actuators, for example, one on each side of the clamp, configured to open the clamp when they are simultaneously actuated. This further reduces the risk of accidental catheter removals.

As the clamp is closed, a force or pressure is applied on the umbilical cord that keeps the umbilical catheter at the proper fixing distance, i.e., it prevents the catheter from accidentally penetrating or pulling out. In addition, once the clamp is closed, the exposed part of the umbilical cord remains inside the clamp protected from possible bacteria, thus reducing the risk of infection.

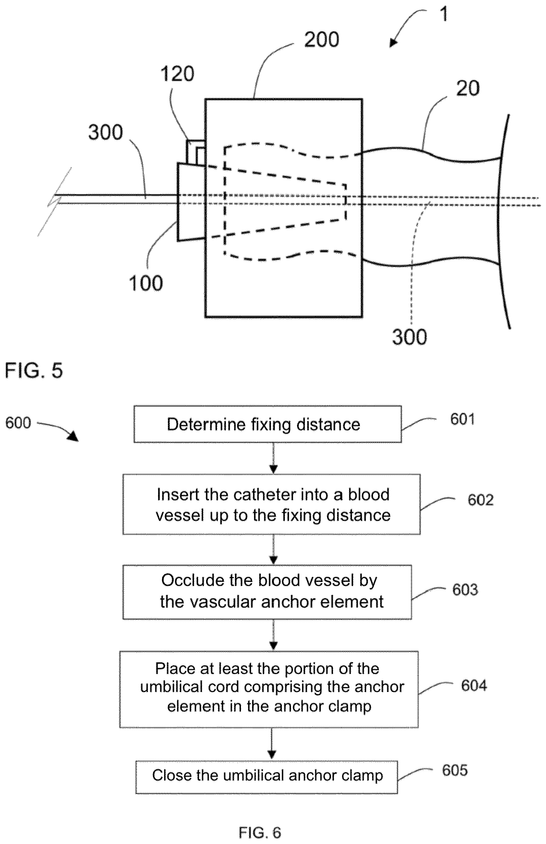

The anchor clamp 200 may further comprise a recess 250, 260 configured to receive at least one portion of the umbilical cord and the at least a portion of the anchor element (see figure 5). The recess 250, 260 may have any shape that facilitates receiving at least one portion of the umbilical cord comprising an anchor element, either a venous or arterial anchor element. In this way the anchor clamp is more versatile as it allows for both venous and arterial cannulation. In one example, the recess may be concave in shape.

Occasionally, it may be necessary to simultaneously cannulating two blood vessels, i.e. two catheters. In such cases, two anchor elements may be used, one for each catheter. In such examples, the recess 250, 260 may be configured to receive a portion of the umbilical catheter comprising two anchor elements. That is, the recess may be configured to receive an umbilical cord with two anchor elements, one for each catheter.

In some examples (not shown), the recess 250, 260 may comprise two slots or two sub-recesses, with each slot being configured to receive a portion of the umbilical cord and a specific vascular anchor element. That is, one slot may be configured to receive the portion of the cord comprising the venous anchor element while the other slot may be configured to receive the portion of the cord comprising the arterial anchor element. In these examples, the same anchor clamp can serve to both cannulation of a single blood vessel and simultaneously cannulation of two blood vessels, increasing the versatility thereof.

In some examples, the anchor clamp 200 may comprise a recess 250, 260 according to any of the examples described in each portion 201, 202.

Additionally, the anchor clamp 200 may comprise a fixing groove 280 configured to removably receive the lug 120 of the vascular anchor element 100 and thereby providing additional fixing to that provided by the anchor clamp portions. The fixing groove 280 may have an internal shape complementary to the lug external shape.

The anchor clamp may comprise an additional fixing element (not shown) on the outside of one of the portions 201, 202. The additional fixing element may be configured to receive or collect a portion of the catheter located outside the patient. This prevents accidental snagging and increases safety. In one example, the clamping element may be fork-shaped. In one example, the anchor clamp may comprise two or more additional fixing elements.

In some examples, the anchor clamp and/or the fixing element may be manufactured from a latex-free medical or paediatric material to avoid allergic reactions. In addition, the material may be any material that allows both parts to be sterilized in order to reduce the risk of infection.

The anchor clamp may be similar in size to a Barr clamp so that the size of the system is as compact as possible to avoid discomfort and accidental tugging. In some examples, the clamp (or the portions) may comprise a length of about 4 - 8 cm. In some examples, the clamp may comprise a length of about 5 - 6 cm. In some examples, the clamp may comprise a width of about 1 - 4 cm. In some examples, the clamp may comprise a width of about 1.5 - 2 cm.

Figure 5 shows in a simplified, schematic form an anchor system 1 according to any of the described examples positioned to anchor an umbilical catheter 300 to the umbilical cord 20.

Figure 6 shows a method 600 for anchoring an umbilical catheter using an anchor system according to any of the described examples.

In use, prior to performing an umbilical cannulation, the catheter fixing distance, i.e., the length or portion of the catheter that will remain within the patient, may be determined at block 601. It is important to determine the appropriate fixing distance for each case in order for the treatment to be effective and to avoid harming the patient.

For example, if the catheter is inserted too far and accidentally rubs or penetrates an organ, it may cause serious sequelae. For determining the appropriate distance for each case, the patient's weight may be taken as a reference and a number of pre-established medical tables relating to weight and fixing distance may be consulted. In some examples, the treatment to be performed and/or the type of blood vessel to be cannulated may also be taken into account in determining the fixing distance.

After severing the umbilical cord, the healthcare personnel may proceed to cannulation of the blood vessel suitable for the treatment, i.e. a vein or an artery. The umbilical catheter may then be inserted, at block 602, up to the determined fixing distance.

In some examples, prior to insertion into catheter, the fixing distance may be measured on the catheter, for example, with a length measuring instrument, such as, for example, a measuring tape or graduated ruler. In some examples, the catheter may comprise a plurality of markings indicating different fixing distances, in such cases the appropriate marking may be taken as an indicator. Healthcare personnel may position their fingers to mark the fixing distance, i.e., the point which separates the portion of the catheter to be inserted and the portion to be left outside the body. Cannulation may be then performed, inserting the catheter up to the indicated point and, once the catheter is inserted, the vascular anchor element may be slid along the catheter until it is inserted into the blood vessel. The blood vessel may then be occluded, at block 603, by the vascular anchor element.

In some alternative examples, prior to inserting the catheter into the blood vessel, the vascular anchor element may be positioned by the healthcare personnel to mark or indicate the fixing distance, for example, by sliding across the catheter. In this way, the vascular anchor element may be used as an indicator or stop, facilitating the cannulation operation and avoiding deviations, thus preventing recannulation. Therefore, multiple radiographs, i.e. repeated radiation exposures, are avoided for checking the correct positioning of the catheter.

In some examples, the umbilical catheter may comprise a plurality of markings, each marking representing a fixing distance to facilitate placement of the anchor element at a particular distance.

In some examples, an X-ray or ultrasound may be performed as a method for double-checking the positioning of the catheter.

Once the vascular anchor element is inserted into the blood vessel, occluding it, at least the portion of the umbilical cord comprising the anchor element may be placed in the anchor clamp at block 604, for example, in a recess located at one of the portions. Additionally, the lug of the vascular anchor element may be coupled to the anchor clamp, for example, to a fixing groove, for enhanced clamping.

Finally, the umbilical anchor clamp may be closed, at block 605, for example, by means of a releasable closure system for anchoring the catheter in position, i.e., at the fixing distance. With the pressure applied by the anchor clamp when closing, the risk that the fixing distance may be accidentally changed, i.e., the catheter may penetrate or it may be pulled out, can be avoided or substantially reduced. In addition, both the blood vessels that have not been cannulated and the exposed portion of the umbilical cord may be occluded or closed, thus reducing the risk of contamination and therefore infection. On the other hand, suturing or tying a silk thread at the base of the cord is avoided, providing greater effectiveness in preventing bleeding and avoiding the use of sharp tools, such that it is safer.

At the end of the treatment, the anchor clamp may be opened and the catheter may be removed, for example, together with the vascular anchor element.

Although only a number of examples have been disclosed herein, other alternatives, modifications, uses and/or equivalents thereof are possible. Furthermore, all possible combinations of the described examples are also covered. Thus, the scope of the present disclosure should not be limited by the particular example but should be determined only by a fair reading of the claims that follow. Reference signs related to drawings placed in parentheses in a claim are solely for attempting to increase the intelligibility of the claim and shall not be construed as limiting the scope of the claim.

## Claims

1. Anchor system for anchoring an umbilical catheter to an umbilical cord, **characterized in that** it comprises:
a vascular anchor element comprising a conduit configured to accommodate a portion of an umbilical catheter, and one end configured to be inserted into a blood vessel of the umbilical cord; and
an anchor clamp configured to releasably retain at least one portion of the umbilical cord and at least one portion of the anchor element, the anchor clamp comprising
a recess configured to receive the at least one portion of the umbilical cord and the at least one portion of the anchor element; and
a releasable closure system for selectively opening/closing the anchor clamp.

2. Anchor system as claimed in claim 1, wherein the anchor clamp comprises two portions hingedly joined together.

3. Anchor system as claimed in claim 1 or 2, wherein the anchor element comprises a lug configured to removably engage an anchor clamp fixing groove.

4. Anchor system as claimed in claim 3, wherein the internal shape of the fixing groove is complementary to the external shape of the lug of the anchor element.

5. Anchor system as claimed in any one of claims 1 to 4, wherein the conduit of the anchor element is configured to allow relative sliding between the catheter and the anchor element.

6. Anchor system as claimed in any of claims 1 to 5, wherein the outer surface of the vascular anchor element is funnel-shaped to facilitate insertion of the anchor element and occlusion of the blood vessel once inserted therein.

7. Anchor system as claimed in any of claims 2 to 6, wherein the releasable closure system comprises:
at least one tab arranged on one of the anchor clamp portions, and
a closure groove located at the opposite portion of the anchor clamp, the groove being configured to releasably receive the at least one tab.

8. Anchor system as claimed in claim 7, wherein the releasable closure system further comprises an actuator for releasing the at least one tab from the closure groove.

9. Anchor system as claimed in any of claims 1 to 8, wherein the end of the vascular anchor element configured to be inserted into an umbilical cord blood vessel comprises an antiseptic coating on the conduit and/or the outer surface to reduce the risk of infection.

10. Anchor system as claimed in claim 9, wherein the coating is in the form of a ring.

11. Anchor system as claimed in any of claims 1 to 10, wherein the anchor element and the anchor clamp are a unitary piece.

12. Anchor system as claimed in any one of claims 1 to 11, wherein the anchor clamp comprises an additional fixing element on the outer surface of the anchor clamp.
